# EUROPÄISCHE PATENTSCHRIFT

(11) **EP 1 049 501 B1**
(45) Veröffentlichungstag und Bekanntmachung des Hinweises auf die Patenterteilung: **22.09.2004**
(21) Anmeldenummer: 98946299.9
(22) Anmeldetag: 07.08.1998
(51) Int. Cl.: A61M 5/20, A61M 5/32

(54) **INJEKTIONSGERÄT**
INJECTION DEVICE
APPAREIL D'INJECTION

(30) Priorität: 24.01.1998 DE 29801168 U
(43) Veröffentlichungstag der Anmeldung: 08.11.2000
(60) Teilanmeldung: 04013434.8
(73) Patentinhaber: Haselmeier S.à r.l., 1295 Mies (CH)
(72) Erfinder: GABRIEL, Jochen, D-70192 Stuttgart (DE)
(74) Vertreter: Raible, Hans, Dipl.-Ing.
(86) Internationale Anmeldenummer: PCT/EP1998/005015
(87) Internationale Veröffentlichungsnummer: WO 1999/037343

(56) Entgegenhaltungen:
- EP-A- 0 114 145
- EP-A- 0 666 084
- WO-A-96/32974
- DE-C- 902 776
- FR-A- 505 931
- FR-A- 1 014 881

## Beschreibung

Die Erfindung betrifft ein Injektionsgerät mit einem Gehäuse und mit einer Energiespeicherfeder zum Speichern von Energie für einen Injektionsvorgang. Diese Energie dient bevorzugt zum automatischen Einstechen einer Injektionsnadel, und ggf. auch zur automatischen Injektion einer Injektionsflüssigkeit. Bevorzugtes Anwendungsgebiet der Erfindung ist ein Injektionsgerät zum einmaligen Gebrauch, oft auch als Einwegspritze bezeichnet.

Aus der EP-A2, 0 666 084 kennt man ein Injektionsgerät dieser Art. Dieses besteht aus zwei Teilen: Einem wiederverwendbaren ersten Teil mit einer Energiespeicherfeder und mechanischen Elementen für die Steuerung des Ablaufs der Injektion, und einem zweiten Teil mit der Injektionsflüssigkeit und der Injektionsnadel. Der zweite Teil wird nach einer Injektion weggeworfen und durch einen neuen Teil ersetzt. Er enthält auch eine Nadelschutzhülse, die nach dem Ende einer Injektion die Nadel überdeckt, um eine Kontamination durch die Nadel zu vermeiden.

Aufgabe der Erfindung ist es, ein neues Injektionsgerät bereitzustellen.

Nach der Erfindung wird diese Aufgabe gelöst durch den Gegenstand des Anspruchs 1. Man erhält so in einfacher Weise einen sequentiellen Ablauf des Injektionsvorgangs, d.h. zuerst wird die Injektionsnadel (Kanüle) in den Patienten eingestochen, und erst anschließend, wenn sich die Nadel bereits im Unterhaut-Fettgewebe befindet, wird der Wirkstoff, der sich im Injektionsgerät befindet, injiziert.
Eine bevorzugte Weiterbildung der Erfindung ergibt sich durch den Gegenstand des Anspruchs 8. Dadurch, dass proximale und distale Endstellung der Nadelschutzhülse eine Funktion der Stellung des Behältercontainers und damit des Verschiebeglieds sind, lassen sich diese Endstellungen optimal an die Bedürfnisse vor und nach einer Injektion anpassen.

Weitere Einzelheiten und vorteilhafte Weiterbildungen der Erfindung ergeben sich aus dem im folgenden beschriebenen und in der Zeichnung dargestellten, in keiner Weise als Einschränkung der Erfindung zu verstehenden Ausführungsbeispiel, sowie aus den Unteransprüchen. Es zeigt:
- Fig. 1: eine Darstellung eines erfindungsgemäßen Injektionsgeräts im Längsschnitt und in seiner gespannten Stellung, also der Stellung vor einer Injektion, und in vergrößertem Maßstab; in der Realität hat das in Fig. 1 dargestellte Gerät z.B. eine Länge von etwa 18 cm und hat etwa die Form eines überdimensionierten Füllfederhalters,
- Fig. 2: eine Draufsicht auf die in Fig. 1 weggebrochen dargestellte Stelle, gesehen in Richtung des Pfeiles II der Fig. 1,
- Fig. 3: eine stärker vergrößerte Darstellung der oberen Hälfte des Injektionsgeräts der Fig. 1, in der gespannten Stellung des Geräts, also vor einem Injektionsvorgang,
- Fig. 4: eine Darstellung analog Fig. 3, aber nach Auslösung eines Injektionsvorgangs, wobei jedoch nur die Nadel eingestochen ist, jedoch noch keine Injektion erfolgt ist,
- Fig. 5: eine Darstellung analog den Fig. 3 und 4, aber nach vollständigem Abschluß einer Injektion,
- Fig. 6 bis 8: schematische Darstellungen zur Erläuterung des sequentiellen Ablaufs einer Injektion,
- Fig. 9: eine Darstellung des proximalen Teils des Injektors vor der Abnahme der Nadelabdeckkappe, welche die Kanüle steril abdeckt,
- Fig. 10: eine raumbildliche Darstellung zum besseren Verständnis der Fig. 9,
- Fig. 11: eine raumbildliche Darstellung des proximalen Endabschnitts der Nadelschutzhülse,
- Fig. 12: eine Darstellung des proximalen Teils des Injektors bei der Abnahme der Nadelabdeckkappe,
- Fig. 13: eine raumbildliche Darstellung zum besseren Verständnis der Fig. 12,
- Fig. 14: eine Darstellung des proximalen Teils des Injektors nach dem Einstechen der Nadel in das Unterhaut-Fettgewebe des Patienten,
- Fig. 15: eine Darstellung des proximalen Teils des Injektors nach dem Herausziehen der Nadel; diese ist hierbei von der Nadelschutzhülse rundum umgeben, um zu verhindern, daß jemand durch die Nadel verletzt oder mit einer Krankheit infiziert wird,
- Fig. 16: eine Draufsicht auf eine an der Nadelschutzhülse vorgesehene Anordnung von Widerhaken,
- Fig. 17: einen Längsschnitt, gesehen längs der Linie XVII-XVII der Fig. 16,
- Fig. 18: eine schematische Darstellung der Widerhakenanordnung vor deren Wirksamwerden, und
- Fig. 19: eine schematische Darstellung der Widerhakenanordnung nach deren Wirksamwerden.

In der folgenden Beschreibung werden die Begriffe proximal und distal in der in der Medizin üblichen Weise verwendet, also: Proximal = dem Patienten zugewandt (die Seite des Injektionsgeräts mit der Injektionsnadel 18); distal = vom Patienten abgewandt.

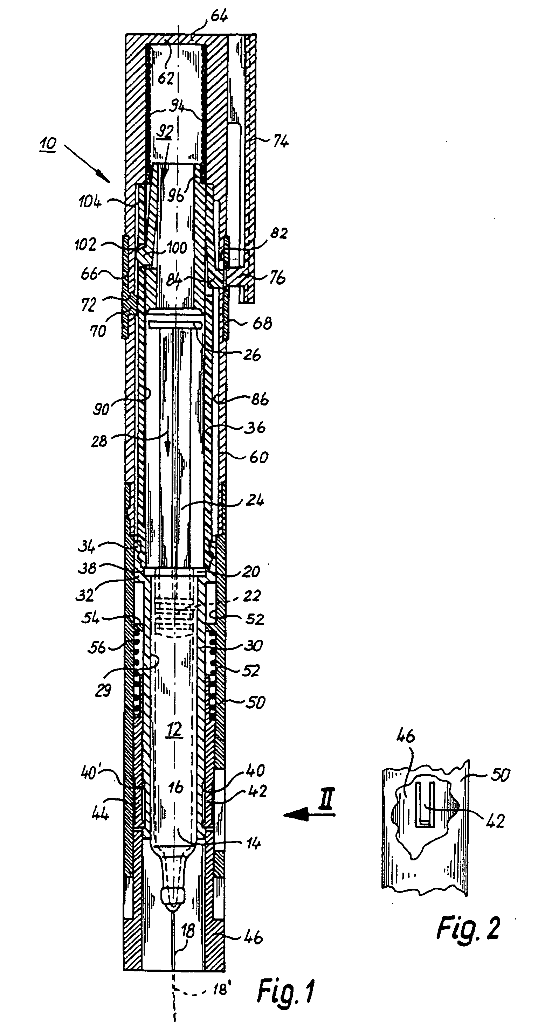

Fig. 1 zeigt die Gesamtheit eines Injektionsgeräts 10 im Längsschnitt. Beim Ausführungsbeispiel ist dies ein Injektionsgerät zum einmaligen Gebrauch, auch Autoinjektor genannt, wobei aber die Erfindung auch bei Injektionsgeräten Anwendung finden kann, die eine mehrfache Verwendung gestatten. Im Inneren des Injektionsgeräts 10 befindet sich bei dieser Ausführungsform eine Injektionsspritze 12 handelsüblicher Bauart, mit einem zylindrischenTeil 14 zur Aufnahme der Injektionsflüssigkeit 16, an dessen proximalem Ende in der üblichen Weise eine Injektionsnadel 18 befestigt ist.

Der zylindrische Teil 14 hat oben in der üblichen Weise eine Verbreiterung 20 in Form von sogenannten Spritzenflügeln. Ferner ist ein Kolben 22 vorgesehen, der mit einer Kolbenstange 24 verbunden ist, die an ihrem distalen Ende eine Druckplatte 26 hat. Drückt man in Richtung des Pfeiles 28 auf die Druckplatte 26, so wird die Flüssigkeit 16 durch die Nadel 18 ausgepreßt, wie das dem Fachmann bekannt ist.

Der zylindrische Teil 14 der handelsüblichen Spritze 12 befindet sich in der zylindrischen Ausnehmung 29 eines Behältercontainers 30, der auch als Spritzencontainer bezeichnet werden kann und der an seinem distalen Endbereich eine Schulter 32 hat, gegen deren distale Seite die Verbreiterung 20 in der dargestellten Weise anliegt. Die Schulter 32 geht über in einen kragenförmigen Abschnitt 34, der in der dargestellten Weise fest mit einem im wesentlichen zylindrisch ausgebildeten Verschiebeglied 36 verbunden ist, welches mit seinem proximalen Ende 38 die Spritzenflügel 20 einklemmt, so daß diese fest mit dem Verschiebeglied 36 und dem Behältercontainer 30 verbunden sind und die Spritze 12 zwangsweise deren Bewegungen folgt.

Der Behältercontainer 30 hat im proximalen Endbereich zwei Nuten oder Ausnehmungen 40, 40', welche einander diametral gegenüberliegen. Eine Nadelschutzhülse 46 hat zwei federnde Abschnitte 42, 44, jeder mit einem radial nach innen ragenden Vorsprung 42', 44' an seinem freien Ende. Der Vorsprung 42' ragt in die Nut 40, der Vorsprung 44' in die Nut 40'. Fig. 2 zeigt den federnden Abschnitt 42 in der Draufsicht.

Auf diese Weise ist die Nadelschutzhülse 46 zwischen einer proximalen und einer distalen Endstellung verschiebbar, deren Abstand durch die (identische) Länge der Nuten 40, 40' bestimmt ist. Verschiebt sich bei der Injektion der Behältercontainer 30 in proximaler Richtung, so ändert sich auch die Lage der Nuten 40, 40', und dadurch die proximale und die distale Endstellung der Nadelschutzhülse 46, wie das nachfolgend ausführlich beschrieben wird, d.h. beide Endstellungen verschieben sich dann in proximaler Richtung. Die Nuten 40, 40' bewirken auch eine Längsführung der Nadelschutzhülse 46.

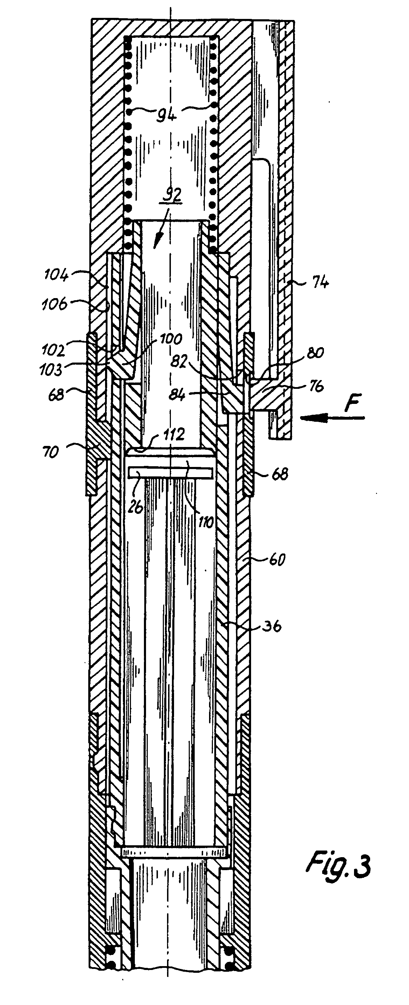

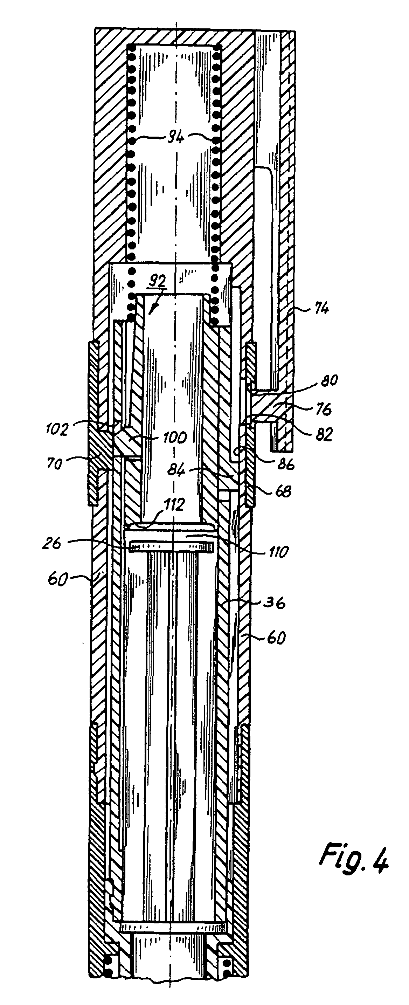

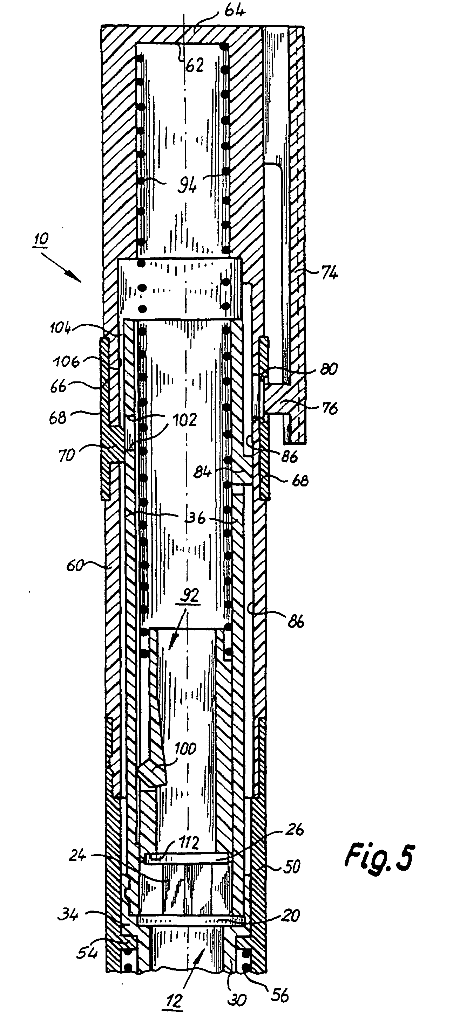

Die Nadelschutzhülse 46 ist gleitend in der zylindrischen Innenseite 52 eines proximalen Gehäuseteils 50 verschiebbar. Von der zylindrischen Innenseite 52 ragt eine Ringschulter 54 radial nach innen. Diese dient als Widerlager für eine Druckfeder 56, welche in der dargestellten Weise die Nadelschutzhülse 46 in proximaler Richtung beaufschlagt, also in Richtung zum Patienten hin. In der zylindrischen Innenseite 52 ist in der dargestellten Weise auch der kragenförmige Abschnitt 34 verschiebbar, und zwar von seiner in Fig. 1 und 3 dargestellten distalen Endstellung bis zu seiner in Fig. 4 und 5 dargestellten proximalen Endstellung, in welcher der Abschnitt 34 gegen die Ringschulter 54 anliegt.

Mit dem proximalen Gehäuseteil 50 ist ein distales Gehäuseteil 60 in der dargestellten Weise fest verbunden. Letzteres hat einen Innenraum 62, der oben, also am distalen Ende, durch eine Abschlußwand 64 verschlossen ist. Auf der Außenseite des Gehäuseteils 60 befindet sich in einer Ringnut 66 ein verdrehbares Ringteil 68, das einen als Steuerglieddienender Nockenabschnitt 70 hat, der durch eine Durchbrechung 72 in der dargestellten Weise in das Innere des distalen Gehäuseteils 60 hineinragt.

An der Außenseite des distalen Gehäuseteils 60 befindet sich in der dargestellten Weise ein Auslöseglied 74, das etwa die Form des Halteclips eines Füllfederhalters hat. Im Bereich seines freien (proximalen) Endes hat das Auslöseglied 74 einen radial nach innen ragenden Vorsprung 76, der dazu dient, einen Injektionsvorgang auszulösen. Bei Fig. 1 wird das verhindert durch das Ringteil 68, das sich in seiner Sperrstellung befindet und dadurch eine Bewegung des Vorsprungs 76 nach links blockiert. Die Fig. 3 bis 5 zeigen dieses Ringteil 68 in einer Drehstellung, in der es die Auslösung einer Injektion ermöglicht, weil sich dort gegenüber dem Vorsprung 76 eine Ausnehmung 80 des Ringteils 68 befindet, die dann mit einer Ausnehmung 82 des distalen Gehäuseteils 60 fluchtet, welche zusammen mit einem Rastteil 84 eine Rastvorrichtung 82, 84 bildet.
Wie Fig. 1 zeigt, ist im gespannten Zustand in die Ausnehmung 82 das radial nach außen federnde Rastteil 84 eingerastet, das hier einstückig mit dem Verschiebeglied 36 ausgebildet ist. Diesem Rastteil 84 ist auf der Innenseite des distalen Gehäuseteils 60 eine Längsnut 86 zugeordnet, in der sich das Rastteil 84 beim Injektionsvorgang verschiebt, vgl. die Fig. 4 und 5.

In der zylindrischen Innenseite 90 des Verschiebeglieds 36 ist ein Auspreßglied 92 gleitend verschiebbar angeordnet. Es ist von einer Druckfeder 94 in proximaler Richtung beaufschlagt, welche im gespannten Zustand (Fig. 1 und 3) die Energie speichert, die zur Durchführung eines Injektionsvorgangs erforderlich ist. Die Feder 94 ist in der dargestellten Weise mit ihrem distalen Ende am Gehäuseabschnitt 64 abgestützt, und mit ihrem proximalen Ende an einer Ringschulter 96 des Auspreßglieds 92.

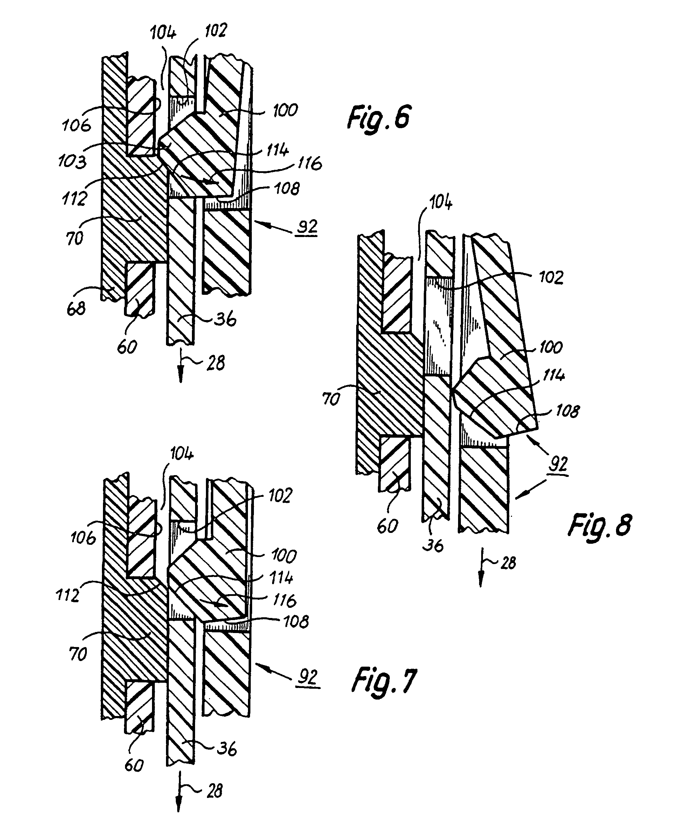

Das Auspreßglied 92 ist einstückig ausgebildet mit einem als federnde Rastnase ausgebildeten elastischen Rastglied 100, dessen Form und Funktion am besten aus den Fig. 6 bis 8 hervorgeht. Im gespannten Zustand des Injektionsgeräts 10 (Fig. 1 und 3) ragt das Rastglied 100 in eine Rastöffnung 102 des Verschiebeglieds 36, und durch diese Ausnehmung 102 ragt es mit einem radialen Überstand 103 radial nach außen in einen radialen Freiraum oder Spalt 104 zwischen Verschiebeglied 36 und Innenseite 106 (Fig. 6 und 7) des distalen Gehäuseteils 60. Dabei stützt es sich mit einer radial verlaufenden Fläche 108 an einer entsprechenden Gegenfläche der Ausnehmung 102 ab, wie in Fig. 6 stark vergrößert dargestellt, so daß die Kraft der Feder 94 über das Rastglied 100 auf das Verschiebeglied 36 übertragen wird und dieses vor Beginn einer Injektion in proximaler Richtung beaufschlagt.

### Arbeitsweise

Zur Auslösung einer Injektion wird in Fig. 3 das Teil 74 durch eine Kraft F beaufschlagt und verschiebt dadurch das federnde Rastglied 84 des Verschiebeglieds 36 radial nach innen, so daß dieses außer Eingriff mit der Ausnehmung 82 des distalen Gehäuseteils 60 kommt.

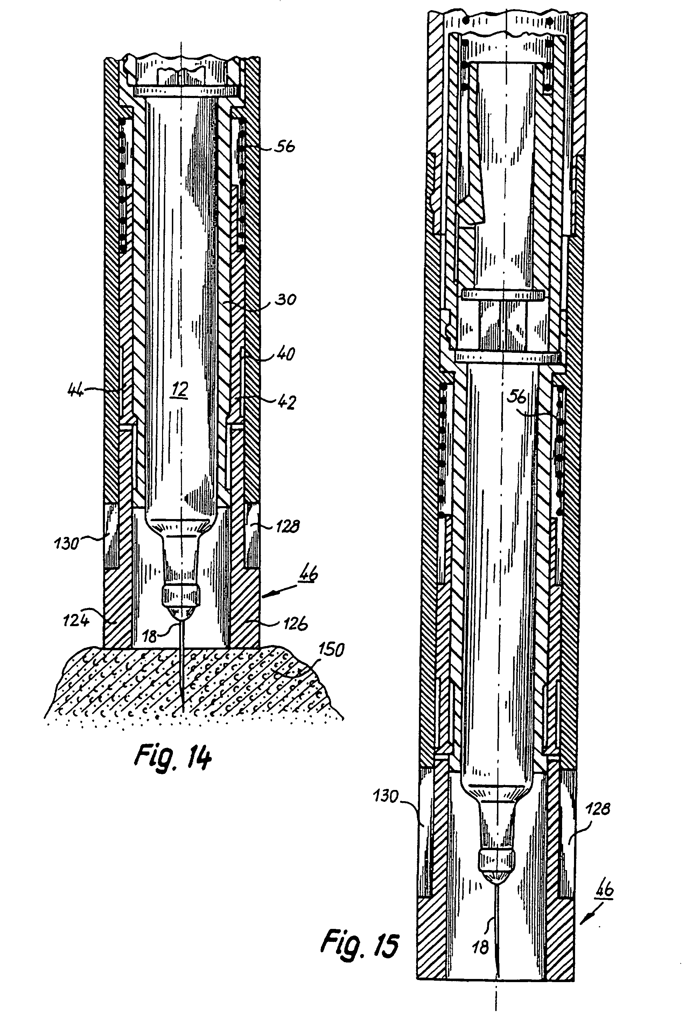

Dadurch können sich gemäß Fig. 4 unter der Wirkung der gespannten Feder 94 Auspreßglied 92 und Verschiebeglied 36 gemeinsam in proximaler Richtung verschieben, da sie durch das elastische Rastglied 100 miteinander gekoppelt sind, und die Nadel 18 wird so in die Stellung verschoben, die in Fig. 1 mit 18' bezeichnet ist, wobei sie in das Unterhaut-Fettgewebe des Patienten einsticht, vgl. Fig. 14.

Gemäß Fig. 4 bleibt hierbei zunächst ein axialer Spalt 110 zwischen dem proximalen Ende 112 des Auspreßglieds 92 und der Druckplatte 26 erhalten, da sich die Spritze 112 synchron mit dem Verschiebeglied 36 bewegt und sich folglich die Lage dieser Teile relativ zueinander nicht verändert. Die Größe des Spalts 110 hängt davon ab, wie groß die Flüssigkeitsmenge 16 in der Spritze 12 ist.

Bei Erreichen der Stellung gemäß Fig. 4 wird das elastische Rastglied 100 durch das Steuerglied 70 radial nach innen ausgelenkt, so daß es außer Eingriff mit der Ausnehmung 102 des Verschiebeglieds 36 gelangt.

Wie dies geschieht, zeigen die Fig. 6 bis 8, die eigentlich keiner Erläuterung bedürfen. Der Vorsprung 70 hat auf seiner distalen Seite eine schräge Fläche 112, der am radialen Überstand 103 eine komplementäre Schrägfläche 114 des elastischen Rastglieds 100 entspricht. Bei einer Bewegung in Richtung des Pfeils 28 gleiten die Schrägflächen 112 und 114 aufeinander und drücken das elastische Rastglied 100 in Richtung eines Pfeiles 116 radial nach innen, so daß es gemäß Fig. 7 außer Eingriff mit der zugeordneten Ausnehmung 102 des Verschiebeglieds 36 gelangt und sich gemäß Fig. 8 unter der Wirkung der Druckfeder 94 selbständig in proximaler Richtung bewegt.

Dabei drückt gemäß Fig. 5 die proximale Stimfläche 112 des Auspreßgfieds 92 gegen die Druckplatte 26 und verschiebt diese bis zum Anschlag in der handelsüblichen Spritze 12, so daß die Flüssigkeit 16 aus dieser ausgepreßt und über die Nadel 18 in den Patienten injiziert wird. Fig. 5 zeigt die Stellung, die nach Abschluß des (automatisch ablaufenden) Injektionsvorgangs erreicht wird.

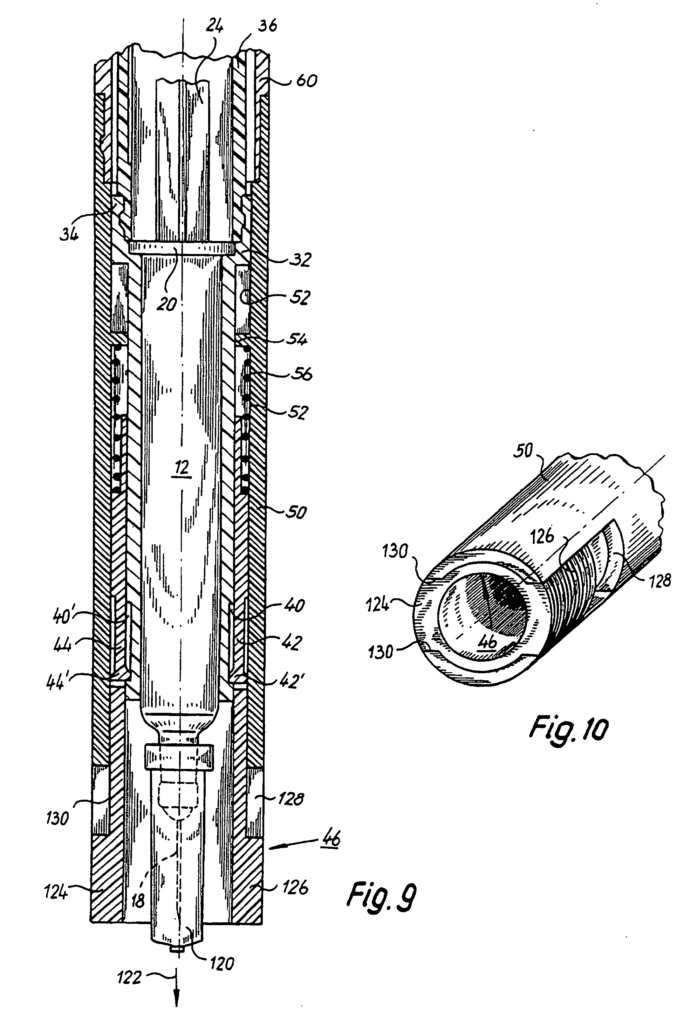

Fig. 9 stimmt mit der Darstellung gemäß Fig. 1 weitgehend überein. Sie zeigt, wie vor einer Injektion eine sterile Nadelabdeckkappe 120 in Richtung eines Pfeiles 122 abgezogen werden muß, damit die Nadel eingestochen werden kann. Im vorliegenden Fall wäre eine Abnahme der Nadelabdeckkappe 120 nur mit Hilfe einer Zange möglich.

Aus diesem Grunde hat die Nadelschutzhülse 46 zwei radiale Vorsprünge 124, 126, mit denen sie in axial verlaufende Aussparungen 128, 130 des proximalen Gehäuseteils 50 ragt und axial in diesen Aussparungen verschiebbar ist.

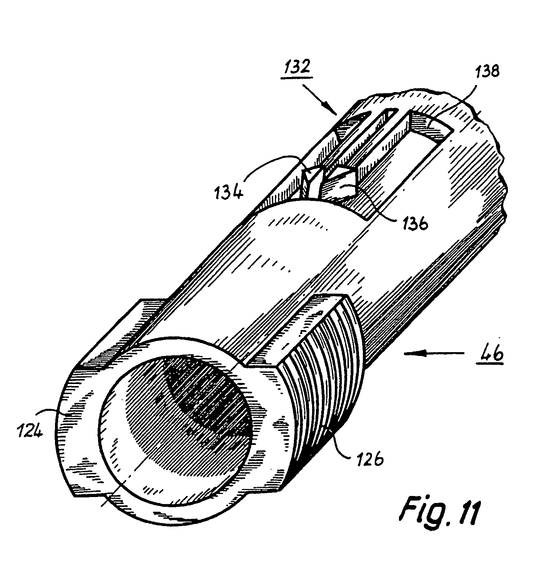

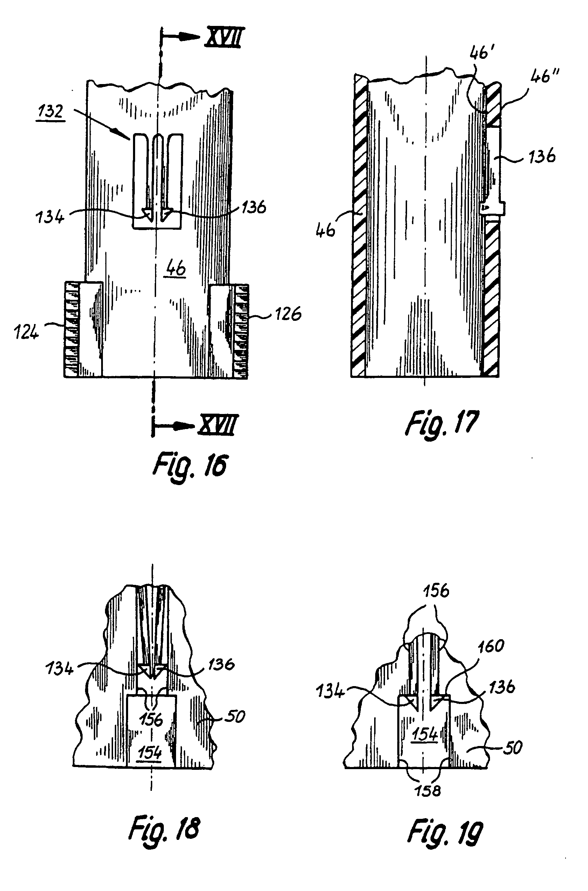

Fig. 11 zeigt in raumbildlicher Darstellung den proximalen Teil der Nadelschutzhülse 46. Diese hat auch eine Rastanordnung 132 mit zwei federnden Widerhaken 134, 136, die sich in einem Fenster 138 befinden. Die Anordnung 132 und ihre Funktion werden nachfolgend erläutert. Die Widerhaken 134, 136 ragen, wie in Fig. 17 klar dargestellt, nach innen und außen radial über den Innenumfang 46' bzw. den Außenumfang 46" der Nadelschutzhülse 46 hinaus. Der äußere Überstand dient zur Führung in einer Längsnut 154 des Gehäuseteils 50, wie in Fig. 18 und 19 dargestellt. Der innere Überstand dient dazu, bei der Montage die Widerhaken 134, 136 in Richtung zueinander auszulenken, vgl. Fig. 18.

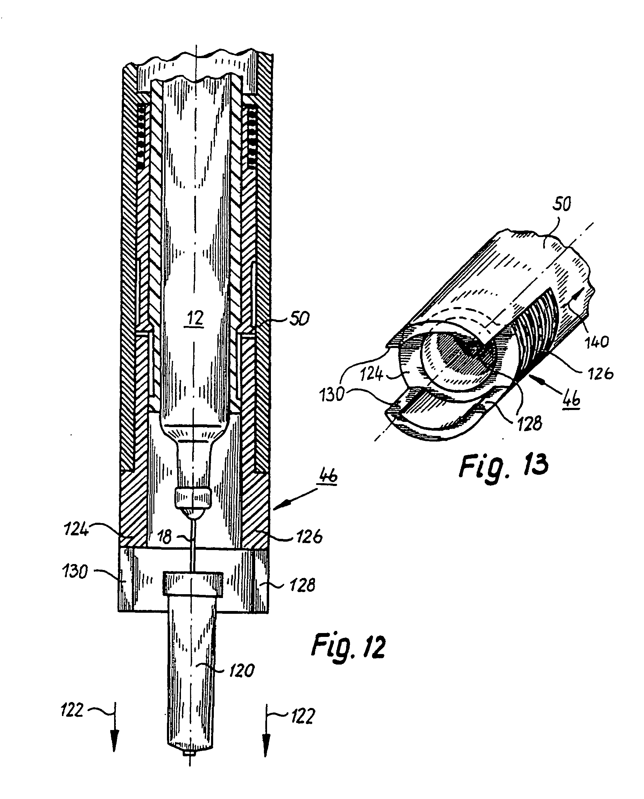

Fig. 12 und 13 zeigen, wie die Nadelschutzhülse 46 in Richtung eines Pfeiles 140 relativ zum Gehäuse 50 in distaler Richtung verschoben worden ist, so daß nun der Patient durch die Aussparungen 128, 130 hindurch die sterile Nadelabdeckkappe 120 packen und in Richtung der Pfeile 122 von der Nadel 18 abziehen kann, um eine Injektion vorzubereiten.

Fig. 14 zeigt die Nadel 18 nach dem Einstechen in das Unterhaut-Fettgewebe 150 des Patienten. Diese Stellung entspricht der Stellung, die in Fig. 4 dargestellt ist (vor dem Injizieren der Flüssigkeit) und ist identisch mit der Stellung, die in Fig. 5 dargestellt ist (nach dem injizieren der Flüssigkeit). Der Unterschied zwischen beiden Figuren liegt in der Stellung des Kolbens 22 im Behälter 14. Dieser Kolben ist in Fig. 14 nicht dargestellt.

Die Nadelschutzhülse 46 hat bei Fig. 14 wieder die Stellung, wie sie in Fig. 9 und 10 dargestellt ist, aber ihre beiden Vorsprünge 42', 44' befinden sich nun am oberen, also distalen Ende der Nuten 40 und 40', weil sich ja der Behältercontainer 30 beim Einstechen der Nadel 18 in proximaler Richtung verschoben hat.

Dadurch hat sich also die distale Endstellung der Nadelschutzhülse 46 entsprechend verändert, und ebenso ihre proximale Endstellung, die, bezogen auf Fig. 14, weiter nach unten gewandert ist.

Wird nun gemäß Fig. 15 die Nadel 18 aus dem Unterhaut-Fettgewebe herausgezogen, so wird die Nadelschutzhülse 46 durch ihre Druckfeder 56 in ihre neue proximale Endstellung verschoben, die in Fig. 15 dargestellt ist und in der sie die Nadel 18 komplett umschließt, um einer Verletzungsgefahr vorzubeugen.

In der Stellung nach Fig. 15 wird die Nadelschutzhülse 46 dauerhaft verrastet, damit sie nicht entgegen der Kraft der Druckfeder 56 versehentlich zurückgeschoben werden kann, was dazu führen würde, daß sich jemand an der Nadel 18 verletzen oder infizieren könnte. Hierzu dienen die beiden Rasthaken 134, 136 der Vorrichtung 132, welche in Fig. 11 raumbildlich dargestellt ist. Diesen Rasthaken ist im Gehäuseteil 50 auf dessen Innenseite eine Längsnut 154 zugeordnet, die in ihrem distalen Bereich 156 schmal ist, so daß dort die Rasthaken 134, 136 zusammengepreßt werden, wie das in Fig. 18 dargestellt ist.

In der Stellung des Geräts 10 gemäß Fig. 15 gelangen, wie in Fig. 19 dargestellt, die Rasthaken 134, 136 in einen breiteren Bereich 158 am proximalen Ende der Nut 154 und rasten deshalb an der Übergangsstelle 160 ein. Dies entspricht der Stellung des Injektors nach Fig. 15, in der die Nadelschutzhülse 46 in ihrer neuen proximalen Endstellung dauerhaft verrastet ist, die damit auch zur - abschließenden - distalen Endstellung geworden ist, wenn das Injektionsgerät - nach Gebrauch - zu Abfall geworden ist.

Mit Ausnahme der Federn 56 und 94 werden die Teile des Injektionsgeräts 10 bevorzugt aus Kunststoff ausgebildet, z.B. aus ABS (Acrylnitril-Butadien-Styrol Polymerisate), PC (Polycarbonat), oder POM (Polyoxymethylen). Bevorzugte Materialien sind:
- Gehäuseteile 50, 60, Nadelschutzhülse 46, Auspreßglied 92 und Verschiebeglied 36: POM oder ABS.
- Behältercontainer 30: POM oder PC.
Die Kunststoffe werden bevorzugt einheitlich ausgewählt, um das Recycling des Injektionsgeräts zu vereinfachen.

Naturgemäß sind im Rahmen der vorliegenden Erfindung vielfache Abwandlungen und Modifikationen möglich.

## Patentansprüche

1. Injektionsgerät mit einem Gehäuse (50, 60) und einer darin angeordneten Energiespeicherfeder (94) zum Speichern von Energie für einen Injektionsvorgang,
mit einem von dieser Energiespeicherfeder in proximaler Richtung beaufschlagten Auspressglied (92) zum Auspressen von Injektionsflüssigkeit (16) aus einem im Gehäuse (50, 60) verschiebbar angeordneten Behälter (14) mit Injektionsflüssigkeit, an dessen proximalem Ende eine Injektionsnadel (18) befestigbar oder befestigt ist,
mit einem im Gehäuse (50, 60) in Längsrichtung verschiebbaren Verschiebeglied (36) zum Verschieben des Behälters (14) im Gehäuse in proximaler Richtung, um eine Bewegung der Injektionsnadel (18) in proximaler Richtung und damit beim Injektionsvorgang ein Einstechen der Injektionsnadel (18) zu bewirken,
mit einer am Auspressglied (92) vorgesehenen *federnden* Rastnase (100), der eine entsprechende Rastöffnung (102) im Verschiebeglied (36) zugeordnet ist, wobei Rastnase (100) und Rastöffnung (102) zusammen ein lösbares Verbindungsglied zwischen Auspressglied (92) und Verschiebeglied (36) bilden,
und mit einem wegeabhängig wirksamen Steuerglied (70) zum Ausrasten der *federnden* Rastnase (100) aus der Rastöffnung (102) dann, wenn das Verschiebeglied (36) beim Injektionsvorgang einen vorgegebenen Weg in proximaler Richtung zurückgelegt hat,
um nach dem Ausrasten der Rastnase (100) eine von einer proximalen Bewegung des Verschiebeglieds (36) unabhängige proximale Bewegung des Auspressglieds (92) und damit ein Auspressen von Injektionsflüssigkeit (16) aus dem Behälter (14) zu bewirken.

2. Injektionsgerät nach Anspruch 1, bei welchem die *federnde* Rastnase (100) im eingerasteten Zustand mit einem radialen Überstand (103) über den Außenumfang des Verschiebeglieds (36) hinausragt,
und durch ein im Verschiebeweg dieses radialen Überstands (103) befindliches Element (70) bei einer proximalen Bewegung des Verschiebeglieds (36) nach innen auslenkbar (Fig. 6 bis 8) und dadurch aus der ihr zugeordneten Rastöffnung (102) ausrastbar ist.

3. Injektionsgerät nach Anspruch 2, bei welchem die *federnde* Rastnase (100) auf ihrer proximalen Seite von innen nach außen gesehen zuerst einen im wesentlichen radial verlaufenden Abschnitt (108) zur Verrastung mit der ihr zugeordneten Rastöffnung (102) und daran anschließend einen schräg verlaufenden Abschnitt (114) aufweist, der, ausgehend vom radial verlaufenden Abschnitt (108), in radialer und distaler Richtung schräg nach außen verläuft.

4. Injektionsgerät nach einem der vorhergehenden Ansprüche, bei welchem zwischen dem Verschiebeglied (36) und dem Gehäuse (50, 60) eine lösbare erste Rastverbindung (82, 84) vorgesehen ist,
welche bei gespannter Energiespeicherfeder (94) eine Verrastung des Verschiebeglieds (36) im Gehäuse (60) ermöglicht,
und welche beim Lösen eine Auslösung eines Injektionsvorgangs bewirkt.

5. Injektionsgerät nach Anspruch 4, bei welchem ein Sperrglied (68) zum Sperren der ersten Rastverbindung (82, 84) vorgesehen ist, um das Auslösen einer Injektion sperrbar zu machen.

6. Injektionsgerät nach einem der vorhergehenden Ansprüche, bei welchem das Auspressglied (92) im inneren des Verschiebeglieds (36) angeordnet und relativ zu diesem in Längsrichtung des injektionsgeräts (10) verschiebbar ist.

7. Injektionsgerät nach einem der vorhergehenden Ansprüche, bei welchem ein Behältercontainer (30) vorgesehen ist, welcher zur festen Aufnahme des Behälters (14) mit Injektionsflüssigkeit dient und welcher mit dem Verschiebeglied (36) fest verbunden ist, so dass Bewegungen des Verschiebeglieds (36) in proximaler oder distaler Richtung über den Behältercontainer (30) auf die Injektionsnadel (18) übertragen werden.

8. Injektionsgerät nach Anspruch 7, bei welchem im Bereich der Injektionsnadel (18) am proximalen Ende des Gehäuses (50, 60) eine Nadelschutzhülse (46) angeordnet ist, welche von einer proximalen Endstellung entgegen der Kraft einer zwischen dem Gehäuse (50, 60) und dieser Nadelschutzhülse (46) wirksamen Feder (56) in eine distale Endstellung verschiebbar ist, wobei proximale und distale Endstellung der Nadelschutzhülse (46) eine Funktion der Stellung des Behältercontainers (30) relativ zum Gehäuse (50, 60) sind.

9. Injektionsgerät nach Anspruch 8, bei welchem die proximale Endstellung der Nadelschutzhülse (46) vor einem Injektionsvorgang im wesentlichen mit der distalen Endstellung der Nadelschutzhülse (46) nach einem Injektionsvorgang übereinstimmt.

10. Injektionsgerät nach Anspruch 8 oder 9, bei welchem zwischen Nadelschutzhülse (46) und Behältercontainer (30) eine mechanische Verbindung (40, 42) vorgesehen ist, welche eine Verschiebung der Nadelschutzhülse (46) relativ zum Behältercontainer (30) innerhalb vorgegebener Grenzen ermöglicht.

11. Injektionsgerät nach einem oder mehreren der Ansprüche 8 bis 10, bei welchem die Nadelschutzhülse (46) eine Rastanordnung (132) aufweist, welche im Bereich einer nach Abschluss eines Injektionsvorgangs durch die - der Nadelschutzhülse zugeordnete - Feder (56) bewirkten proximalen Endstellung der Nadelschutzhülse (46) eine Verrastung mit dem Gehäuse (50) bewirkt, um nach einer Injektion eine Überdeckung der Injektionsnadel (18) durch die Nadelschutzhülse (46) zu bewirken.

## Claims

1. Injection device with a housing (50, 60) and an energy-storing spring (94) arranged therein for storage of energy for an injection operation,
with an expressing member (92) subject to the action of this energy-storing spring in the proximal direction for expressing injection liquid (16) from a container (14) with injection liquid arranged displaceably in the housing (50, 60) and to whose proximal end an injection needle (18) is or can be fastened,
with a displacing member (36) displaceable in the housing (50, 60) in the longitudinal direction for displacing the container (14) in the housing in the proximal direction in order to cause a movement of the injection needle (18) in the proximal direction and hence insertion of the injection needle (18) during the injection operation,
with a resilient detent, lug (100) which is provided on the expressing member (92) and has associated therewith a corresponding detent opening (102) in the displacing member (36), the detent lug (100) and detent opening (102) together forming a releasable connecting member between the expressing member (92) and the displacing member (36),
and with a control member (70) which is effective dependent on distance travelled and serves for disengaging the resilient detent lug (100) from the detent opening (102) when the displacing member (36) has covered a predetermined distance in the proximal direction during the injection operation,
in order to cause a proximal movement of the expressing member (92) independent of a proximal movement of the displacing member (36) after the disengagement of the detent lug (100) and hence expressing of injection liquid (16) from the container (14).

2. Injection device according to claim 1, in which the resilient detent lug (100) projects beyond the external circumference of the displacing member (36) in the engaged state with a radial protrusion (103), and can be deflected inwards by an element (70) located in the displacement path of this radial protrusion (103) during a proximal movement of the displacing member (36) (Figs. 6 to 8) and thereby disengaged from the detent opening (102) associated with it.

3. Injection device according to claim 2, in which viewed from the inside outwards the resilient detent lug (100) comprises on its proximal side firstly a portion (108) which runs essentially radially for engagement with the detent opening (102) associated with it, and after it a portion (114) which runs obliquely and which, starting from the radial portion (108), runs obliquely outwards in the radial and distal directions.

4. Injection device according to one of the preceding claims, in which a releasable first detent connection (82, 84) is provided between the displacing member (36) and the housing (50, 60), which connection allows engagement of the displacing member (36) in the housing (60) when the energy-storing spring (94) is cocked, and initiates an injection operation when released.

5. Injection device according to claim 4, in which a locking member (68) is provided for locking the first detent connection (82, 84) in order to make it possible to prevent the initiation of an injection.

6. Injection device according to one of the preceding claims, in which the expressing member (92) is arranged in the interior of the displacing member (36) and is displaceable relative to the latter in the longitudinal direction of the injection device (10).

7. Injection device according to one of the preceding claims, in which a container holder (30) is provided which serves for secure reception of the container (14) with injection liquid and which is connected securely to the displacing member (36) so that movements of the displacing member (36) in the proximal or distal direction are transmitted through the container holder (30) to the injection needle (18).

8. Injection device according to claim 7, in which there is a needle protection sleeve (46) which is arranged in the region of the injection needle (18) at the proximal end of the housing (50, 60) and which is displaceable from a proximal end position against the force of a spring (56) acting between the housing (50, 60) and this needle protection sleeve (46) into a distal end position, the proximal and distal end positions of the needle protection sleeve (46) being a function of the position of the container holder (30) relative to the housing (50, 60).

9. Injection device according to claim 8, in which the proximal end position of the needle protection sleeve (46) before an injection operation is essentially the same as the distal end position of the needle protection sleeve (46) after an injection operation.

10. Injection device according to claim 8 or 9, in which a mechanical connection (40, 42) is provided between the needle protection sleeve (46) and the container holder (30), allowing displacement of the needle protection sleeve (46) relative to the container holder (30) within predetermined limits.

11. Injection device according to one or more of claims 8 to 10, in which the needle protection sleeve (46) comprises a detent arrangement (132) which causes engagement with the housing (50) in the region of a proximal end position of the needle protection sleeve (46) produced after completion of an injection operation by the spring (56) associated with the needle protection sleeve in order to ensure that the injection needle (18) is covered by the needle protection sleeve (46) after an injection.

## Revendications

1. Appareil d'injection muni d'un boîtier (50, 60) et d'un ressort (94) accumulateur d'énergie, disposé dans ledit boîtier afin d'accumuler de l'énergie nécessaire à un processus d'injection,
comprenant un organe d'expulsion (92), sollicité dans une direction proximale par ce ressort accumulateur d'énergie, en vue d'exprimer du liquide d'injection (16) d'un récipient (14) qui renferme du liquide d'injection, est logé à coulissement dans ledit boîtier (50, 60), et à l'extrémité proximale duquel une aiguille d'injection (18) est ou peut être fixée,
un organe de coulissement (36) pouvant coulisser longitudinalement dans le boîtier (50, 60), en vue de faire coulisser le récipient (14) dans ledit boîtier, dans une direction proximale, de façon à provoquer un mouvement de l'aiguille d'injection (18) dans la direction proximale, et donc un enfoncement de ladite aiguille d'injection (18) au cours du processus d'injection,
un bec élastique (100) à déclic, prévu sur l'organe d'expulsion (92) et auquel est assigné un orifice d'encliquetage (102) correspondant, pratiqué dans l'organe de coulissement (36), le bec à déclic (100) et l'orifice d'encliquetage (102) formant, associativement, un organe de solidarisation dissociable entre l'organe d'expulsion (92) et l'organe de coulissement (36),
et un organe de commande (70) agissant en fonction de la course, pour désencliqueter le bec élastique (100) à déclic hors de l'orifice d'encliquetage (102) lorsque l'organe de coulissement (36) a effectué une course préétablie, dans la direction proximale, lors du processus d'injection,
en vue de provoquer, après le désencliquetage du bec à déclic (100), un mouvement proximal de l'organe d'expulsion (92) indépendant d'un mouvement proximal de l'organe de coulissement (36) et, de ce fait, une expulsion de liquide d'injection (16) hors du récipient (14).

2. Appareil d'injection selon la revendication 1, dans lequel le bec élastique (100) à déclic fait saillie au-delà du pourtour extérieur de l'organe de coulissement (36) à l'état encliqueté, par une zone radiale débordante (103),
et peut être dévié vers l'intérieur (figures 6 à 8) lors d'un mouvement proximal dudit élément de coulissement (36), sous l'action d'un élément (70) situé sur le trajet de coulissement de cette zone radiale débordante (103), de sorte que ledit bec peut être désencliqueté de l'orifice d'encliquetage (102) qui lui est affecté.

3. Appareil d'injection selon la revendication 2, dans lequel le bec élastique (100) à déclic présente sur son côté proximal, observé de l'intérieur vers l'extérieur, tout d'abord une région (108) s'étendant pour l'essentiel dans le sens radial, en vue d'un encliquetage avec l'orifice d'encliquetage (102) qui lui est affecté et, dans l'enchaînement direct, une région inclinée (114) qui s'étend à l'oblique vers l'extérieur, dans les directions radiale et distale, à partir de ladite région (108) s'étendant radialement.

4. Appareil d'injection selon l'une des revendications précédentes, dans lequel est prévue, entre l'organe de coulissement (36) et le boîtier (50, 60), une première solidarisation encliquetée (82, 84) dissociable
qui autorise un encliquetage dudit organe de coulissement (36) dans ledit boîtier (60), lorsque le ressort (94) accumulateur d'énergie est bandé,
et provoque, lors de la dissociation, le déclenchement d'un processus d'injection.

5. Appareil d'injection selon la revendication 4, dans lequel un organe de blocage (68) est prévu pour bloquer la première solidarisation encliquetée (82, 84), afin d'offrir une faculté de blocage du déclenchement d'une injection.

6. Appareil d'injection selon l'une des revendications précédentes, dans lequel l'organe d'expulsion (92) est logé dans l'espace interne de l'organe de coulissement (36) et peut coulisser, vis-à-vis de ce dernier, dans la direction longitudinale dudit appareil d'injection (10).

7. Appareil d'injection selon l'une des revendications précédentes, dans lequel est prévu un réceptacle (30) qui sert à recevoir rigidement le récipient (14) renfermant du liquide d'injection, et est relié rigidement à l'organe de coulissement (36) de façon telle que des mouvements dudit organe de coulissement (36), dans une direction proximale ou distale, soient répercutés sur l'aiguille d'injection (18) par l'intermédiaire dudit réceptacle (30).

8. Appareil d'injection selon la revendication 7, dans lequel une douille (46) protège-aiguille, disposée dans la région de l'aiguille d'injection (18) à l'extrémité proximale du boîtier (50, 60), peut être déplacée d'une position extrême proximale à une position extrême distale, en s'opposant à la force d'un ressort (56) agissant entre ledit boîtier (50, 60) et cette douille (46) protège-aiguille, les positions extrêmes proximale et distale de ladite douille (46) protège-aiguille étant fonction de la position du réceptacle (30) vis-à-vis dudit boîtier (50, 60).

9. Appareil d'injection selon la revendication 8, dans lequel la position extrême proximale de la douille (46) protège-aiguille, avant un processus d'injection, coïncide pour l'essentiel avec la position extrême distale de ladite douille (46) protège-aiguille après un processus d'injection.

10. Appareil d'injection selon la revendication 8 ou 9, dans lequel est prévue, entre la douille (46) protège-aiguille et le réceptacle (30), une solidarisation mécanique (40, 42) autorisant un coulissement de ladite douille (46) protège-aiguille, vis-à-vis dudit réceptacle (30), dans des limites préétablies.

11. Appareil d'injection selon l'une ou plusieurs des revendications 8 à 10, dans lequel la douille (46) protège-aiguille présente un dispositif d'encliquetage (132) qui provoque un encliquetage avec le boîtier (50) dans la région d'une position extrême proximale de ladite douille (46) protège-aiguille due à l'action du ressort (56) - assigné à ladite douille protège-aiguille ― à l'achèvement d'un processus d'injection, afin d'instaurer un recouvrement de l'aiguille d'injection (18), par ladite douille (46) protège-aiguille, après une injection.
